# EUROPEAN PATENT SPECIFICATION

(11) **EP 4 306 049 B1**
(45) Date of publication and mention of the grant of the patent: **29.04.2026**
(21) Application number: 23184859.9
(22) Date of filing: 11.07.2023
(51) Int. Cl.: A61B 5/15, A61B 5/153, A61B 5/155, G16H 10/60, G16H 40/60, G16H 50/20, G16H 40/63, G16H 20/17, G16H 40/67, G16H 70/40, A61M 5/142

(54) **AUTOMATIC DRUG INFUSION MONITORING DEVICE WITH TIMED SAMPLES**
AUTOMATISCHE VORRICHTUNG ZUR ÜBERWACHUNG DER INFUSION VON MEDIKAMENTEN MIT ZEITGESTEUERTEN PROBEN
DISPOSITIF DE SURVEILLANCE AUTOMATIQUE DE PERFUSION DE MÉDICAMENT AVEC ÉCHANTILLONS TEMPORISÉS

(30) Priority: 14.07.2022 US 202263389159 P
(43) Date of publication of application: 17.01.2024
(73) Proprietor: Phlebotics, Inc., West Lafayette, IN 47906 (US)
(72) Inventor: KISSINGER, Candice B., West Lafayette, 47906 (US); KISSINGER, Peter, West Lafayette, 47906 (US)
(74) Representative: reuteler & cie SA

(56) References cited:
- US-A1- 2006 189 926
- US-A1- 2009 069 743
- US-A1- 2010 121 170
- US-A1- 2010 273 738
- US-A1- 2011 313 318

## Description

### FIELD OF DISCLOSURE

The disclosure relates to an apparatus and unclaimed method for use in treating patients and performing biomedical research, and in particular to an apparatus and unclaimed method for collecting blood samples or other fluids from patients for diagnostic or research purposes.

### BACKGROUND

Infusion therapy is a type of treatment which involves infusing fluid or medication (in a solution, for example) via intravenous or subcutaneous injection via a fenestrated catheter attached to an infusion pump. In some aspects, a doctor or nurse who is treating a patient using infusion therapy needs to also draw blood samples from the patient at predetermined periods of time, for example, once every morning, to monitor the patient's condition as well as to observe whether the infusion therapy is working properly and there is no serious physiological response (e.g., side effect) to the treatment. Infusion therapy has a range of medical applications such as sedation, anesthesia, post-operative analgesic pain management, chemotherapy, and treatment of infectious diseases, and its efficacy due to precise medication delivery is one of its advantages over non-site-specific delivery methodologies.

When the patient is undergoing infusion therapy, the doctor or nurse writes down for the record the times of day during which the fluid or medication was infused and during which the blood sample was drawn. However, human errors can result for various reasons. For example, the doctor or nurse may write down the wrong time for the sample taken or infusion performed, write down the time on the records for different patients when there are multiple patients to take care of, neglect to write down the time entirely, "round up" or "round down" the recorded time to the nearest quarter-hour or half-hour to quicken the time-recording process, or even write down vague terms for the time, such as "early morning" or "mid-afternoon" which makes determining the actual time impossible In some situations, the doctor or nurse may neglect to take samples or perform infusion entirely. In any event, such human error, whether involuntary or on purpose, may render analysis of the infusion therapy or monitoring of the patient's health condition difficult or impossible. As such, there is a need to reduce such human error in infusion therapy and treatment.
US2011313318A1 relates to methods and systems for determining the concentration of one or more analytes from a sample such as blood or plasma. The systems described therein can be configured to withdraw a certain volume of sample from a source of bodily fluid, direct a first portion of the withdrawn sample to an analyte monitoring system and return a second portion of the sample to the patient. The analyte monitoring system can include an automated blood withdrawal system that is configured to withdraw blood from the patient's vasculature at low pressure and/or withdrawal rates so as to reduce or prevent contamination of the withdrawn fluid from the infusion fluids.

### SUMMARY

The invention is set forth in claims 1 and 13. Dependent claims recite advantageous embodiments of the invention. The present disclosure provides an apparatus and unclaimed method for automatic drug infusion monitoring device with timed samples. An automated blood sampling and infusion system includes: a sampling pump in communication with a blood sample collection apparatus; an infusion pump configured to perform infusion; and a controller configured to: control the infusion pump to perform the infusion on a patient during an infusion event, control the sampling pump to draw blood from the patient into the blood sample collection apparatus during a sample collection event following the infusion event, and automatically store in a memory unit a timestamped data of the infusion event and the sample collection event. There may be multiple infusion events and sample collection events.

In some examples, the timestamped data includes (a) start time of the sample collection event or the infusion event and (b) end time of the sample collection event or the infusion event. In some examples, the timestamped data further includes an amount of blood drawn during the sample collection event or an amount of fluid solution expelled during the infusion event. The infusion event includes the type and amount of the fluid solution that is expelled.

In some examples, the automated blood sampling and infusion system includes a user interface to input information regarding the sample collection events and the infusion events. In some examples, the blood sample collection apparatus include a plurality of sample containers. Each sample container receives one sample of blood drawn from the patient during one sample collection event, and the sample container is linked to the timestamped data assigned to that sample collection event. In some examples, an amount of blood drawn for the sample ranges from 0.01 cm³ (cubic centimeter, cc) to 10 cm³ (cc). In some examples, a user interface facilitates inputting the amount of blood to be drawn for the sample.

In some examples, the automated blood sampling and infusion system includes at least one additional electronic device operatively coupled with the controller via a wired or wireless network connection, the at least one additional electronic device to provide information regarding the sample collection event and the infusion event and to receive the timestamped data of every sample collection event and infusion event facilitated by the controller.

In some examples, the automated blood sampling and infusion system includes a multi-lumen catheter comprising at least a first lumen and a second lumen, wherein the first lumen is in fluid communication with the infusion solution storage and the second lumen is in fluid communication with the sample collection storage.

In some examples, the memory unit stores therein a drug library comprising instructions on the sample collection events and the infusion events associated with each of a plurality of drugs (or fluid solutions) administered during the infusion event.

In some examples, the controller is configured to control the sampling pump to perform a plurality of sample collection events associated with a plurality of timestamped data. In some examples, the blood sample collection apparatus includes a cassette removably installed therein and containing a plurality of sample containers, and each of the sample containers contains one sample of the blood drawn from the patient during one of the sample collection events.

In some examples, the blood sample collection apparatus is operable to refrigerate the sample containers during the sample collection events. In some examples, an amount of blood drawn for the sample ranges from 0.01 cm³ (cc) to 10 cm³ (cc). In some examples, a user interface facilitates inputting the amount of blood to be drawn for the sample and a time interval between the sample collection events.

According to some embodiments, an automated blood sampling and infusion system includes: a sampling pump in communication with a blood sample collection apparatus containing a plurality of sample containers, each of the sample containers configured to contain one sample of blood drawn from a patient during one of a plurality of sample collection events; an infusion pump configured to perform infusion during a plurality of infusion events; a user interface configured to facilitate inputting an amount of blood to be drawn for each of the samples and a time interval between the sample collection events; and a controller. The controller may be configured to: control the infusion pump to perform the infusion on the patient during one of the infusion events; control, based on the amount of blood to be drawn and the time interval that are inputted, the sampling pump to draw the blood from the patient into the blood sample collection apparatus during one of the sample collection events following the infusion event; and automatically store in a memory unit a timestamped data of the infusion event and the sample collection event. In some examples, the amount of blood drawn for the sample ranges from 0.01 cm³ (cc) to 10 cm³ (cc).

Also disclosed herein are methods of automating blood sampling and infusion. In some examples, the method includes: controlling, by a controller, an infusion pump to perform infusion on a patient during an infusion vent; controlling, by the controller, a sampling pump to draw blood from the patient into a blood sample collection apparatus during a sample collection event following the infusion event, and automatically storing, by the controller in a memory unit, a timestamped data of the infusion event and the sample collection event.

In some examples, the method further includes determining, by the controller, the condition of the patient based on patient condition measurements received from at least one sensor.

In some examples, the timestamped data includes (a) start time of the sample collection event or the infusion event and (b) end time of the sample collection event or the infusion event. In some examples, the timestamped data further includes an amount of blood drawn during the sample collection events or an amount of fluid solution expelled during the infusion events. The infusion event includes the type and amount of the fluid solution that is expelled.

In some examples, the method includes receiving, by the controller via a user interface, information regarding the sample collection events and the infusion events. In some examples, the blood sample collection apparatus include a plurality of sample containers, and the method further includes configuring, by the controller, each sample container to receive one sample of blood drawn from the patient during the sample collection event; and linking, by the controller, the sample container to the timestamped data assigned to the sample collection event. In some examples, the method includes cooling (or maintaining at an optimal temperature) each of the blood samples (or sample containers/vials) to improve or ensure stability of the collected samples.

In some examples, the method further includes receiving, by the controller from at least one additional electronic device operatively coupled with the controller via a wired or wireless network connection, information regarding the sample collection event and the infusion event; and transmitting, by the controller to the at least one additional electronic device, the timestamped data of every sample collection event and infusion event facilitated by the controller.

The above mentioned and other features, and the manner of attaining them, will become more apparent and the invention itself will be better understood by reference to the following description taken in conjunction with the accompanying drawings.

### BRIEF DESCRIPTION OF THE DRAWINGS

The disclosure is explained in greater detail below in reference to the figures. In the figures:
FIG. 1 is a schematic diagram of a fluid sampling and infusion system in accordance with an embodiment;
FIG. 2 is a schematic diagram of the controller and an additional device connected via the network connection from FIG. 1 in accordance with an embodiment;
FIG. 3 is a schematic diagram of the fluid sampling and infusion device of FIG. 1 in accordance with an embodiment;
FIG. 4 is a flowchart of a process as performed by the controller of FIG. 1 in accordance with an embodiment;
FIG. 5 is a flowchart of a process as performed by the controller of FIG. 1 in accordance with an embodiment;
FIG. 6 is a perspective view of an automated blood sampling and infusion system in accordance with an embodiment;
FIG. 7 is a perspective view of an automated blood sampling and infusion system in accordance with an embodiment; and
FIGs. 8A through 8C are partial views of the automated blood sampling and infusion system during different phases of an automated blood sampling procedure in accordance with an embodiment.

### DETAILED DESCRIPTION

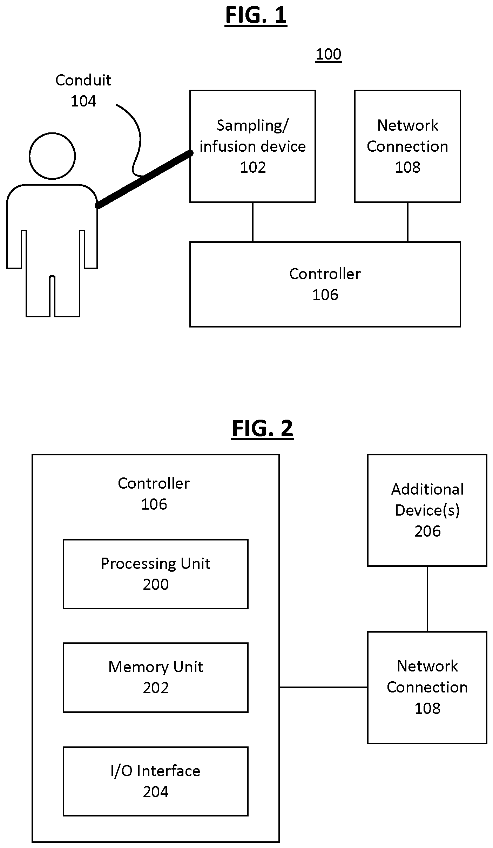

Referring to FIG. 1, an automated fluid sampling and infusion system 100 is shown according to some embodiments. The system 100 includes a device 102 for fluid sampling and infusion. The fluid that is sampled from the patient as referred to herein is blood, but other types of bodily fluids such as urine or cerebrospinal fluid, for example, may be sampled in some cases. The device 102 may be a single device or multiple devices, each with a specific functionality different from the others, that operate together (e.g., in an integrated implementation) for blood sampling or infusion. The device 102 is connected to a conduit 104 such as one or more catheters which collect blood samples from the patient and provide drug/fluid solution (such as treatment solution) to the patient's body for infusion. There may be multiple conduits, each having its own function of collecting blood samples or performing infusion in the treatment. The device 102 may be sealed to prevent contamination of the fluid inside (whether it is the sample collected or the solution to be used for infusion) from external factors.

In some embodiments, there may be a single vascular access for both infusion and blood collection. In such cases, the conduit 104 may be a single-lumen or multi-lumen catheter. Prior-art methods as known in the art involve repurposing the original infusion catheter for sampling by manually advancing an inner catheter or internal flow tube through a peripheral intravenous (PIV) line placed through the skin of a patient such that the inner catheter is advanced beyond the tip of the PIV line and into the blood vessel to collect a blood sample. After blood collection, the inner catheter may be manually retracted and removed from the PIV line to be discarded. An example of such method may utilize one or more PIVO^{™} device from Velano Vascular, a division of Becton, Dickinson and Company. These manual approaches are very labor-intensive and may open the catheter to the atmosphere with a potential risk of nosocomial infection. In comparison, a multi-lumen catheter (defined as a catheter having a minimum of two lumens therein, where one lumen is used to introduce the therapeutic fluid and the other lumen is used to withdraw blood from the patient) helps facilitate automatic infusion and blood collection without manual human interaction by doctors or physicians, for example. The multi-lumen catheter may have two ports open to the bloodstream, such that a distal (or downstream) port delivers the infusion and a proximal (upstream) port is accessible by the automated blood sampler. In such examples, the vascular access may be configured in this manner to preclude sampling blood immediately downstream from the infusion, before the drug has passed through the circulation, since doing so may provide a sample with an inaccurate drug concentration. In some embodiments, confusion may be avoided by color-coding the extravascular connectors and/or by using different mechanical connection formats for drug infusion and blood sampling, for example. Therefore, in some examples, the conduit 104 may be a multi-lumen catheter with at least a first lumen and a second lumen, such that the first lumen is in fluid communication with the infusion solution storage and the second lumen is in fluid communication with the sample collection storage.

The system 100 includes a controller 106 operatively coupled with the sampling and infusion device 102 or with the subcomponents thereof, as further explained herein. The controller 106 controls the operation of the device 102 based on user input or predefined setting. The controller 106 may also be capable of making its own decisions, independent of user input, regarding the timing of blood sampling or infusion, or the type, amount, and/or concentration of fluid to be provided for infusion, as further explained herein. A network connection 108 may be provided for the controller 106 in some examples such that the controller 106 is operatively connected with a network such as a computer network or digital communications network, as known in the art.

FIG. 2 shows an example of the controller 106 in some implementations. The controller 106 may be a computing device such as a desktop computer, laptop computer, tablet computer, smartphone, or any other suitable device capable of controlling the device 102 for blood sampling and infusion as programmed. The controller 106 includes a processing unit 200 which may be a processor such as a general-purpose processor, a special-purpose processor, a conventional processor, a digital signal processor (DSP), a plurality of microprocessors, one or more microprocessors in association with a DSP core, a microcontroller, Application Specific Integrated Circuits (ASICs), Field Programmable Gate Array (FPGAs) circuits, any other type of integrated circuit (IC), a state machine, and the like. The processing unit 200 may perform signal coding, data processing, power control, input/output processing, and/or any other functionality that enables the device 102 to operate as intended by the user. The controller 106 may be coupled to the device 102 via wire or wirelessly.

The controller 106 further includes a memory unit 202 which may be any suitable memory, such as non-removable memory such as random-access memory (RAM), read-only memory (ROM), a hard disk, or any other type of memory storage device. Additionally or alternatively, the suitable memory may be removable memory such as a subscriber identity module (SIM) card, a memory stick, a secure digital (SD) memory card, and the like. Additionally or alternatively, the processing unit 200 may access information from, and store data in, memory that is not physically located near the device 102 but is remote, such as on a server or a separately located computer, operatively coupled via the network connection 108. The memory unit 202 may be any suitable tangible or non-transitory computer-readable medium capable of storing thereon instructions as one or more computer programs or algorithms, for example, which causes the processing unit 200 to perform certain methods or procedures, as further disclosed herein.

The controller 106 further includes an input/output interface 204 such as an interactive input/output device including but not limited to a touchscreen display, interactive physical or virtual buttons, and/or microphone/speaker combination, to name a few. The interface 204 is configured to receive information from the user as well as to display information to be viewed by the user. The collection of blood sample is referred to as a "sample collection event," and the facilitation of infusion in the patient's body is referred to as an "infusion event." The sample collection event and the infusion event may each be determined via user inputs or predetermined settings of the system 100, for example. The interface 204 may be a graphical user interface (GUI) displayed on a monitor, a display, or a touchscreen device, for example.

The system 100 may include one or more additional devices 206 which are connected with the controller 106 via the network connection 108 to send data to and/or receive data from the controller 106, for example via a cloud network, Internet, or any other suitable form of digital data communication.

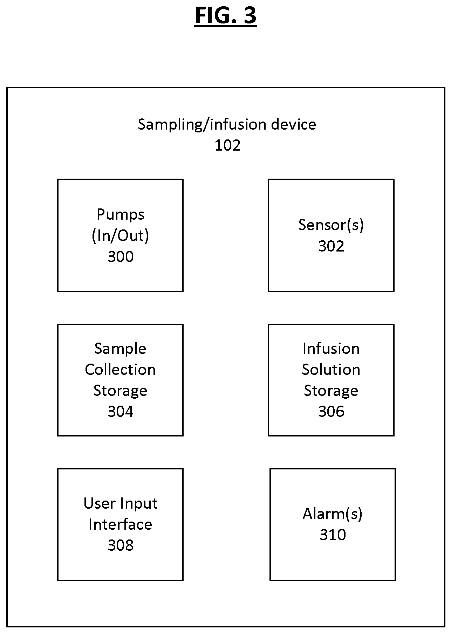

FIG. 3 shows the components of the device 102 for blood sampling and infusion, according to some implementations. The device 102 includes pumps 300 capable of collecting fluid (e.g., blood) for sampling from the patient in an inflow and also providing fluid (e.g., drug solution) for infusion in an outflow. One or more of the pumps may be designed for inflow and/or outflow. The pumps may each be assigned a specific role, such as one that is specifically for infusion (infusion pump) and another specifically for sample collection (collection pump or sampling pump). For example, the infusion pump may be unidirectional, and the sampling pump may be bidirectional. The infusion pump may operate for a longer period of time (for example, at least 1 hour at a time or longer, such as 2 hours or more) and the sampling pump may operate for a shorter period of time (for example, cycling for collection events that each typically lasts about 1 to 5 minutes).

In some examples, the device 102 also includes one or more sensors 302 capable of detecting conditions of the patient and/or the blood sample collected from the patient. For example, a pressure sensor may be implemented to measure blood pressure of the patient, a thermometer may be implemented to measure body temperature of the patient, as well as any other suitable sensor known in the art. The sensors 302 may also be capable of analyzing the blood sample taken from the patient. For example, after the blood sample is collected, the sensors 302 may automatically measure and analyze the sample to provide a complete blood count (CBC) test, such that any number that is out of the ordinary (e.g., reduced red blood cell count, white blood cell count, platelet count, etc.) may be flagged by the device 102 or by the controller 106 coupled to the device 102, as suitable. In some examples, the sample analysis result is used by the controller 106 to recommend a dosage of the infusion fluid in the next or subsequent infusion event.

The device 102 includes a sample collection storage 304 and an infusion solution storage 306. The sample collection storage 304 may include a plurality of containers (such as vials, resealable bags, etc.), and each container (e.g., vial) receives one sample of the fluid drawn from the patient during the sample collection event. The container may be sealed after the appropriate sample is taken to prevent the sample from being contaminated. The infusion solution storage 306 may include one or more types of fluid to be expelled to be used for infusion, such as drug solution or saline solution, for example, where each type of fluid is separated from each other in different containers to prevent intermixing or contamination of fluids. In some examples, in order to improve or ensure stability of the collected samples, the sample collection storage 304 or the containers contained therein may be cooled or maintained at an optimal temperature (for example, using any suitable air conditioning unit or temperature control device) after collecting blood samples. Other environmental parameters such as pressure and/or humidity, for example, may also be controlled as suitable to optimize the stability of the samples.

In some examples, the different samples stored in the storage 304 during the sample collection events as well as the amounts and types of fluid being expelled from the storage 306 during the infusion events are automatically timestamped by the processing unit 200 of the controller 106 in real time as the device 102 operates, and recorded or stored in the memory unit 202 of the controller 106. In some examples, the controller 106 may be implemented as part of the device 102 or as a separate modular device that may be disconnected from the device 102. In some examples, the device 102 includes its own processing unit and/or memory unit separate from the controller 106, and the processing unit of the device 102 may timestamp and store the sample data in its memory unit. In some examples, the data may be stored in a remote database connected to the device 102 via a cloud network or Internet-of-things (IoT) infrastructure.

In some examples, the device 102 includes a user input interface 308 which may be similar to or different from the interface 204 of the controller 106. For example, the user input interface 308 may be one or more buttons that can be pressed by the user to begin the sample collection event or the infusion event, without having to rely on the controller 106. In some examples, the user input interface 308 is an interactive display such as touchscreen panel. In some examples, the user input interface 308 may have more limited and/or simplistic functionality than the interface 204 of the controller 106 so as to prevent the user from erroneously entering a different setting or starting the wrong event, thereby providing a safety mechanism against the user possibly overdosing the patient, infusing the patient with a wrong solution, or taking too many blood samples for the patient which may be unnecessary or even harmful to the patient (including but not limited to a patient who is very young or neonatal/newborn patient, a patient who has a small body type, or a patient who is prone to having anemia).

In some examples, the device 102 includes one or more alarms 310 which provide sensory or auditory alerts or notifications in response to detecting certain conditions. For example, the conditions may include abnormal measurement in the blood sample, possible occlusion or air detected in the conduit 104, or the device 102 not operating as it is intended, among others. In the latter case, the conditions may include the conduit or conduits 104 being detached from the patient or any component of the device 102 (such as the pumps 300, sensors 302, storages 304 and 306, or interface 308) malfunctioning or disconnected, either physically or electrically. In some examples, the alarms 310 may indicate a low battery status of the device 102, or the device 102 being disconnected from a power source. The alarm 310 may be auditory (e.g., a voice explaining the situation or a beeping sound to alert the user) and/or visual (e.g., colored alert sign when certain conditions are detected, otherwise green or yellow when a low-risk condition is detected, for example).

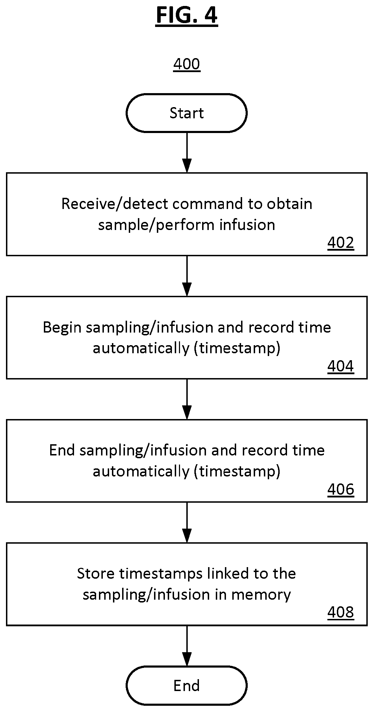

FIG. 4 shows a process 400 which may be implemented by the controller 106 in some embodiments. The process 400 may be facilitated by the processing unit 200 of the controller 106 when the processing unit 200 runs a computer program code or instructions stored on the memory unit 202 or more specifically the non-transitory computer-readable medium. In step 402, the processing unit 200 receives or detects a command to obtain a sample from or to perform infusion on the patient.

In step 404, the processing unit begins the sampling or infusion by operating the pumps of the associated sampling and infusion device such that the device collects the blood sample from the patient (in a sample collection event) and/or expels a fluid solution to the patient for infusion (in an infusion event), as suitable. The exact time of each event is recorded automatically (i.e., timestamped), and the sample taken during the sample collection event is associated with the specific timestamp as recorded. Similarly, the infusion time is also recorded automatically, such that each timestamped infusion event is associated with a specific value of fluid dispelled from the device (e.g., a type of fluid, an amount or volume of the fluid, and a concentration of the fluid, etc.).

In step 406, the processing unit 200 ends the sampling or infusion by pausing or stopping the operation of the pump(s) as suitable, and the time at which the pump(s) is paused or stopped (or the time at which the sampling or infusion is ended) is also automatically recorded or timestamped as the end of the sample collection event or the infusion event, as suitable. The timestamp data is then stored in the memory in step 408, and the sampling or infusion event associated with the timestamp data is also linked to the timestamp in the memory.

The timestamp may thus be automatically facilitated such that a first timestamp indicates a start time of an event and a second timestamp indicates an end time of an event. In some examples, the timestamp may span a period of time instead of marking an instance of time, such that the beginning of a timestamp indicates the start time of the event, and the end of the timestamp indicates the end time of the event.

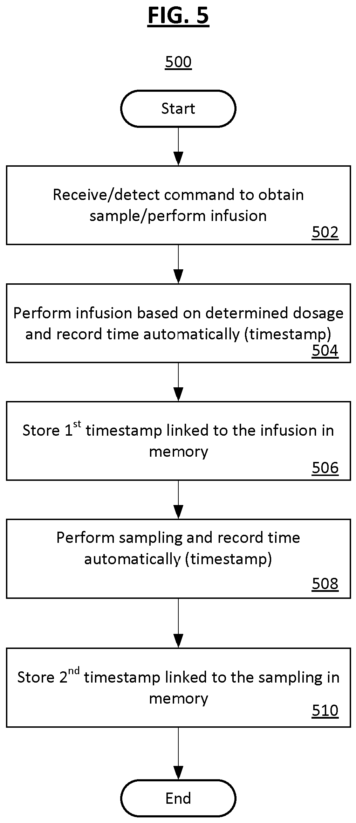

FIG. 5 shows another process 500 which may be implemented by the controller 106 in some embodiments. In step 502, the processing unit of the controller receives or detects a command to obtain a blood sample from the patient and to perform infusion on the patient, as suitable. In step 504, the infusion is performed in an infusion event, and the exact time of the infusion event is recorded as a first timestamp. In step 506, the first timestamp is stored in the memory, and the first timestamp is linked to the infusion event.

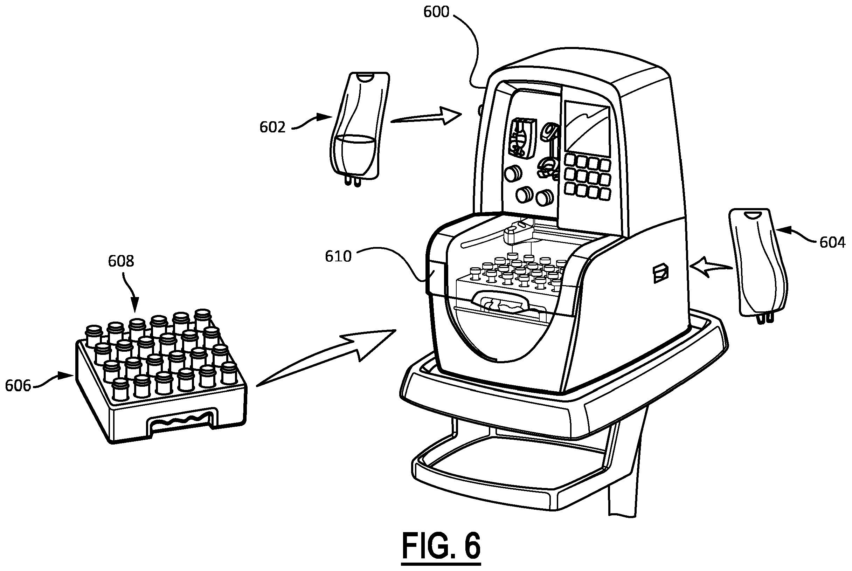

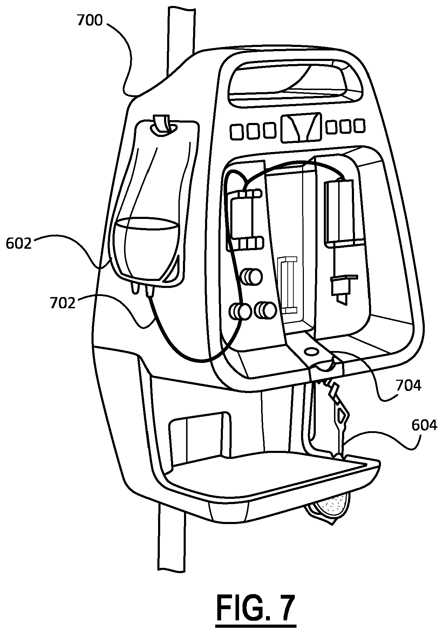

FIG. 6 shows an apparatus 600 as used in the automated fluid sampling and infusion system 100 according to some embodiments. The apparatus 600 may be referred to as an automated fluid sampling and infusion device with different device components which can be removably attached thereto or installed therein. For example, a sterile saline packet 602 provides the sterile saline solution to allow for the flushing of any catheter(s) or tube(s) that is used for fluid sampling during an interval between two different sampling events, such as the catheter/tube 702 shown in FIG. 7, for maintenance purposes. The sterile saline packet 602 may be attached to or installed on one side of the apparatus 600 while a waste saline packet 604, which receives excess blood from the patient and/or the saline solution after flushing the apparatus, may be attached to or installed on the other side. The apparatus 600 may also receive a vial cassette 606 (which may also be referred to as a blood sample collection apparatus) which may be removably installed in the apparatus 600 and may house, hold, or contain multiple vials 608 or sample containers for sampling the bodily fluid (for example, blood) of a patient. Saline solution may flow from the packet 602 into the apparatus 600 to flush the catheter or tube periodically, such as into the patient's body, and any remaining fluid or particles in the apparatus may be transported into the waste saline packet 604 for easy disposal at a later time. This flushing may be repeated to reduce the risk of contamination between samples and/or to avoid the clogging up of the catheter or tube between sampling events. The cassette 606 makes it easier for multiple vials 608 to be installed and removed simultaneously without the risk of accidentally rearranging the order of the vials 608, as the vials 608 may be arranged such that each slot of the cassette 606 represents a specific sample taken at a specific time. The apparatus 600 houses the processing unit 200 and the memory unit 202 as well as providing the I/O interface 204 as shown in FIG. 2. The apparatus 600 may also be referred to as the sampling/infusion device 102 as shown in FIG. 1 and thus may contain any one or more of the components as shown in FIG. 3 according to some examples. Also, the apparatus 600 may maintain the samples in refrigeration by providing a casing 610 which can be closed when the samples (e.g., the cassette 606 holding the vials 608 containing the samples) are stored inside the apparatus 600. In some examples, the cassette 606 may facilitate the refrigeration or retain the samples in a refrigerated state. Although not shown in FIG. 6, the apparatus 600 may also have an infusion device or component integrated or incorporated therewith, or the infusion device or component may be a separate device(s) that is integrated into the overall system 100 and is operable in conjunction with the apparatus 600, in order to facilitate the infusion events in addition to the sample collection events as mentioned above.

FIG. 7 shows another apparatus 700 as used in the automated fluid sampling and infusion system 100 according to some embodiments. The packets 602 and 604 are attached or hooked to the side of the apparatus 700 while fluidly coupled with the appropriate components within the apparatus 700 through catheters or tubes 702, for example. As explained above, the saline solution from packet 602 may be used to clean the inside channel or lumen of the catheters or tubes 702 after each sample collection event. The apparatus 700 is designed so as to automatically maintain integrity of the catheters or tubes 702. The catheter or tube 702 may be multiple catheters or tubes operating independently of one another, or a single catheter or tube with multiple lumens or channels that are independently operable, for example. The apparatus 700 also includes a slot or holder 704 for the vials 608 to be used for collecting blood samples. The apparatuses 600 and 700 are designed to be programmable to serially draw blood sample(s) in a predetermined or programmed time interval or schedule and in a predetermined or programmed volume for each sample. The amount of blood to be drawn and/or the time interval may be inputted via a user interface such as the interface 204 or 308 as explained above. The apparatuses 600 and 700 may be battery operated and may be mobile (such as being attached to a stand with rollers to facilitate moving to different locations within a hospital or other places, as suitable). The apparatuses 600 and 700 may be auto-maintained (that is, maintained automatically to require minimal maintenance by the users) and may be compatible with all suitable diagnostic equipment as known in the art.

In some examples, the apparatuses 600 and 700 may be manually triggered by a caregiver or research technician (for example, in a drug clinical trial). In some examples, such as in the apparatus 600, there may be provision for refrigerating the collected samples, such as by providing an electrically powered refrigeration system or device, which may be powered by a battery or any other suitable type of energy supply associated with the apparatus. In some examples, such as in the apparatus 700, the collection vials may be manually inserted and/or removed. The aforementioned apparatuses may be suitably modified, for example, to add or remove any one or more of the aforementioned components or capabilities, in order to reduce the cost of manufacturing the apparatuses, to reduce the overall weight or dimensions of the apparatuses, and/or to increase the energy efficiency (such as battery life and consumption) of the apparatuses.

Refrigerating the samples assists in slowing down chemical reactions in the sample collected. Some of the chemical reactions include, but are not limited to, enzymatic reactions, decomposition of certain drugs, oxidation by dissolved oxygen, and so on. Refrigerating the samples further assists in slowing down the clotting cascade (or gradual clotting of the samples collected), and the samples that are constantly refrigerated have more time to mix with anticoagulants which may be used to coat the interior of the sampling tube (such as the conduit 104 of FIG. 1 and the catheters or tubes 702 of FIG. 7, for example). Therefore, leaving the samples at room temperature before mixing is complete may cause the clotting process to begin. In a typical manual sampling process, the nurse or phlebotomist may gently tip the tubes back and forth about 8-10 times after the sample is collected in order to assure that the anticoagulant is sufficiently mixed, especially when using larger tubes where the diffusion distance is greater between the dissolving anticoagulant and the freshly collected blood sample. Neglecting to tip the tubes (e.g., if the caregiver, nurse, or phlebotomist is distracted in a busy hospital environment) may cause the aforementioned clotting, thereby causing problems with the diagnostic instruments which use the collected samples for analysis. For example, the formation of such clots may distort the measured results especially in hematology instruments. Incorrect results may pose a potential threat to the safety of the patients even if the error occurs relatively infrequently. The additional step of the aforementioned manual "mixing by tipping" may not be reasonably feasible when a series of samples are to be collected based on a predetermined schedule over several hours. Furthermore, handling the sampling tube too aggressively (for example by manually pulling against the tube) may cause the samples to experience hemolysis, which may necessitate resampling to maintain the integrity of the analysis of the samples, causing further delay in providing critical data for the patient. As such, it is beneficial to constantly refrigerate the samples after collection.

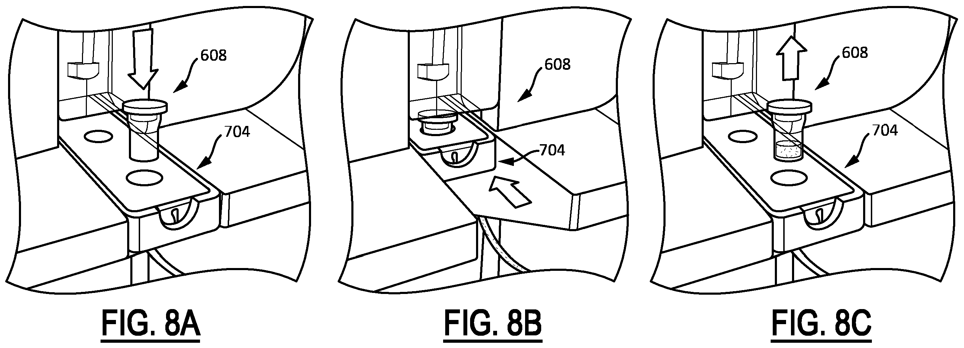

FIGs. 8A through 8C show different steps in a fluid-drawing process as implemented by the apparatus 700. In FIG. 8A, an empty vial 608 is placed (e.g., downwardly as indicated by the arrow) on the holder 704 while the holder 704 is located externally. In FIG. 8B, the holder 704 holding the empty vial 608 is inserted into the apparatus 700 as shown by the inwardly pointing arrow for fluid sample collecting (or any other type of bodily fluid, as appropriate). In FIG. 8C, the holder 704 returns to the initial state as in FIG. 8A, but the vial 608 is now filled with a predetermined or programmed amount of blood sample to be retrieved from the holder 704 (e.g., upwardly as indicated by the arrow) to be replaced with a new empty vial 608 to repeat the process for collecting multiple samples. The vials 608 may be of any suitable size and shape, and in some cases multiple vial formats may be implemented to distinguish between different types of samples. Each vial 608 may only contain a single sample obtained during a single sample collection event. In some examples, the sample collection may be automatic, such that the insertion of the holder 704 into the apparatus 700 may be sufficient to trigger the sample collection. In some examples, the sample collection is manually activated, such as by pressing a button to collect a sample of a predetermined or programmed amount.

Beneficially, the system 100 facilitates automated blood drawing such that the number of blood-draws required can be preprogrammed, and the amount of blood that is drawn can be small, such as less than 0.1 cm³ (cc), less than 0.05 cm³ (cc), less than 0.04 cm³ (cc), less than 0.03 cm³ (cc), less than 0.02 cm³ (cc), or any other suitable range or value therebetween. In some examples, the amount of each sample may be less than a drop, e.g., less than 0.025 cm³ (cc) and even as low as 0.01 cm³ (cc) (or 10 µL). Alternatively, the amount may range to several cm³ (cc), for example up to 1 cm³ (cc), up to 5 cm³ (cc), up to 10 cm³ (cc), or any other suitable range or value therebetween, as preprogrammed by the user. Because all the values are programmed, the system 100 has high precision regarding the volume and timing of each sampling, such as by precisely controlling the sampling amount and the sampling interval.

In some cases, the precision in time and allowing the volume of sample collecting to be small may be important for patients who may not be physically capable of providing a high volume of bodily fluid such as blood for sampling. For example, pediatric venipuncture volumes is a topic of constant discussion because the amount of blood that can be safely removed from a child may vary depending on the size and age of the child, such as a newborn. In some examples, the guidelines as set forth in CLSI GP41-Ed7 indicate that, assuming 75-80 mL/kg blood volume in children (or higher in newborns), blood collection from a child should be limited to 1-5% of total blood volume over 24 hours and also to 10% total blood volume over 8 weeks. Furthermore, the European Commission Guideline indicates that, assuming 80-90 mL/kg blood volume in children, the recommended blood collection volume from a child should be limited to 1% of total blood volume over 24 hours and also to 3% total blood volume over 4 weeks, although the guideline indicates that the recommendations are not evidence-based. Other institutions may have different values as part of the guidelines implemented in the institutions, so it is difficult to determine the upper threshold levels of how much blood may be safely collected from children. Furthermore, there is generally reluctance to draw blood from young children, so if the samples are collected manually, such samples may be collected less frequently such as only once a day or once a week, thereby resulting in insufficient number of samples for proper analysis. Therefore, it is preferred to take as little as possible in terms of blood samples from such patients, especially newborns, as well as other patients who many have a small body mass or may be susceptible to anemia, etc., while still maintaining the necessary frequency and interval in which the samples are being taken. As such, the system 100 benefits from automated blood sample collecting by minimizing the sample volume and maintaining a predetermined or preprogrammed sampling frequency so as to make it easier for doctors and physicians to determine a pattern or trend in the collected blood samples in order to make their diagnosis or to monitor the prognosis.

In some examples, a doctor or nurse may determine the patient's health condition based on the collected sample data and/or sensor measurements from a previous cycle, or from additional/external devices that are operatively coupled with the processing unit. For example, the doctor or nurse may analyze using the appropriate sensor measurements whether the patient is suffering any medical condition that may require immediate medical attention or a change in the infusion dosage. Based on the determined condition, the doctor or nurse may determine the dosage of infusion accordingly to be implemented in the infusion step 504 (e.g., type, amount/volume, and/or concentration of the drug for infusion). In some examples, the processing unit may recommend a dosage of the infusion fluid in the next or subsequent infusion event based on a result of an analysis performed by the sensors on the collected sample, such as a CBC test, which may be accepted or rejected by the doctor or nurse. In step 504, the infusion is facilitated by the processing unit by controlling the pumps to limit the dosage of infusion to the dosage determined by the doctor or nurse. The exact time of the infusion (for example, beginning and ending times of the infusion event) is automatically recorded and timestamped as the first timestamp, as are the information pertaining to the dosage given during this event.

**The** determined dosage may include the specific amount or volume of the solution to be used for infusion, as well as the concentration of the solution that is being used. In some examples, the pumps may operate such that at least one pump is connected to the infusion solution storage of the infusion device and at least one pump is connected to a different storage which stores a solution used to dilute the infusion solution provided by the infusion solution storage. That is, when the concentration of the infusion fluid is to be reduced, the pump connected to the diluting solution may be activated as suitable to dilute the infusion fluid by mixing the diluting solution with the infusion fluid.

In step 508, the processing unit may operate the pumps (for example, the sampling pump) to perform sampling, such that predetermined amount or volume of the patient's blood can be collected as a sample at a predetermined time, and subsequently stored. The time of the sampling event is recorded (timestamped) automatically. In step 510, a second timestamp for the sampling event is linked to the data associated with the collected sample and stored in the memory unit. The process 500 may be restarted when another command is detected for the next sample collection event and infusion event.

In some examples, the timestamped data is organized in a table (for example, a pivot table or lookup table) or an illustrated chart for visibility when the users access and review the data, which may facilitate improved decision making by the doctors or nurses. In some examples, the command may be provided as a set of timed instructions (e.g., each of the sample collection events and infusion events have a specified time or margin of time in which they must be performed) or as a manual user input such as when a button is pressed or actuated.

In some examples, the sample collection and infusion device is a smart device which is capable of communicating with other devices such as smartphones or computers, as well as with other similar devices as suitable, such as in an IoT setting. In such cases, the smart device, which includes its own processor and memory, may store in its memory (e.g., memory unit 202) a drug library which is a list of drugs which is used as reference by the processor of the smart device in order to prevent medication errors. In some embodiments, the drug library may include protocols or instructions for commonly infused drugs that define the flow rate and total volume reflecting the mass of drug (for example, in mg) to be dosed over a defined time (for example, in seconds or minutes). Individual drugs may be cleared from circulation at widely different rates on average, and these rates of clearance may vary substantially from one patient to another. The exposure of a patient to a drug is often defined as the area under the curve (AUC) of a graph depicting drug concentration-versus-time. With concentration data available, the exposure to the drug can be adjusted in subsequent infusions. In some examples, if an antibiotic dose results in a concentration that is too low, that is, below the minimum inhibitory concentration (MIC) for the invading microbe, the efficacy may be inadequate. On the other hand, if the concentration is too high, the patient may be subject to toxicity in specific organs such as the kidneys, liver, or heart, etc. Clearance (and thus concentration and AUC) varies with patient size, age, gender, types of co-administered drugs, and organ function capabilities (including, but not limited to, kidney and liver function, for example).

Once the population of subpopulation averages are known from clinical trials, such data may be used to determine a dose recommendation in the prescribing information provided by the drug manufacturer and approved by the Food and Drug Administration (FDA). A mathematical model of the pharmacokinetics may also provide the expectations for clearance and AUC. Alternative or additionally, the dynamics for the average patient may suggest a specific post-dose blood sampling protocol sequence. In some embodiments, a few points (for example, between 3 to 5 points) of blood sampling at specific times may sufficiently enable dose adjustment for the individual patient. Such schedule may vary from one drug to another and may be programmed into the controller 106 to facilitate the automatic blood sampling and infusion, or more specifically, programmed into the drug library which the controller 106 utilizes. Therefore, in some examples, the drug library may include protocols or instructions on the sample collection event and the infusion event associated with each of a plurality of drugs administered during the infusion event. Alternatively, such protocol may be programmed flexibly via an external port to a network, such as via the network connection 108, using the additional device(s) 206.

For example, each drug may set one or more parameters such as concentration, infusion rate, and dosage thresholds (maximum and minimum), and the doctor or nurse may be required to confirm the correct drug and dosage to implement for each patient (if there are multiple patients) manually via the aforementioned user interface. In some examples, there are different levels of dosage thresholds. For example, a first level is a preferred dosage amount or dosage range which the doctor or nurse may exceed under certain circumstances. A second level may be an absolute dosage limit which may never be exceeded under any circumstance, since doing so increases the risk of the patient undergoing complications from the drug. In some examples, the different levels of dosage thresholds may be determined in view of the combination of drugs that are to be delivered. In some examples, the different levels of dosage thresholds may be determined using the patient's physical data such as age, weight, gender, etc. In some examples, the different levels of dosage thresholds may also be determined by the smart device using the patient's past or historical data such as medical history. In some examples, the smart device may retrieve such data from an external database via the network connection.

By implementing the above, the smart device may intercept errors such as incorrect dosage rate, dosage amount, and/or pump settings. Other benefits include reducing the chances of adverse drug events (e.g., overdose or underdose), improving the doctors and nurses' practice, and increasing the cost effectiveness of the system.

In some examples, the smart device may be capable of approximating and subsequently adjusting drug dosage such as the dosage rate, dosage amount, and/or pump settings based on sensor measurement during clinical trials or clinical applications/treatments. For example, initial drug dosage parameters (which may be defined as including any one or more of the dosage rate, dosage amount, and/or pump setting for the particular drug) may be established based on the patient's size (height and/or weight), age, gender, body mass index (BMI), and any applicable preexisting condition such as those indicated in the medical record. The drug dose parameters are determined and stored as a first approximation, for example in the memory unit of the device. During the clinical trial or application, the drug concentration within the patient's body varies with time. Since the blood volume varies among different patients as does the drug's rate of clearance from blood circulation, the smart device in some examples may take one or more sample measurements of the blood volume or rate of clearance (using any sensor suitable for such purpose) and calculate subsequent drug dosage parameters based on such measurements. For example, after taking such measurements, the device may calculate the next dosage parameters by adjusting the previously stored dosage parameters based on the measurements. That is, a second set of dosage parameters may be determined by adjusting the initial (first set of) dosage parameters, and a third set of dosage parameters may be determined by adjusting the second set of dosage parameters. With each cycle of adjustment, the approximation of the dosage parameters will be more precise than before, thereby providing a process of calculating the best dosage parameters that are suitable for the patient, or patient-specific, taking into account different factors such as the variations in drug absorption and excretion (related to metabolism, transporters, and organ function integrated over time) and/or the qualitative and quantitative variations in drug target (receptor) expressions, for example. In some examples, in order to prevent overdosing, the initial dosage parameters may be set at low threshold values, and the adjustments gradually increase the parameter values to reach the best dosage parameters. The adjustment may be based on, for example, comparing the drug concentration measurement with a threshold concentration value (which may be predetermined or learned via machine learning using tens, hundreds, or thousands of datapoints, as known in the art) or using a lookup table pertaining to the specific type or class of drug that is administered, as stored in the memory unit.

The drugs may be grouped or categorized in the system based on what "type" they are associated with. Some of the types of drugs which may be used for this purpose may include, but are not limited to: immunosuppressants, antibiotics, anticoagulants, insulins, chemotherapeutics, and so on. Each type has its own threshold or region of efficacy as well as its threshold or region of safety. For example, immunosuppressants may lead to organ rejection if the dosage is too low, but toxicity or infections may take over if the dosage is too high. Antibiotics may not be capable of inhibiting bacterial growth if the dosage is too low, but they may be toxic if the dosage is too high. Insulins may cause hyperglycemia if the dosage is too low, but they may cause hypoglycemia if the dosage is too high. Chemotherapeutics may fail to rid the body of cancer cells if the dosage is too low, but they may destroy other healthy cells as well if the dosage is too high. The search for a "Goldilocks zone" of these types of drugs, therefore, is an ongoing battle for doctors and patients alike, and in some cases, overdosing and underdosing can be easily prevented if human errors (including but not limited to errors in the dosage parameters as well as in the sampling, such as missing a sampling period or mistakenly inputting one sample value as another sample value in a different sampling time period, for example) or dosage data loss (that is, missing data due to data file corruption or misplacement of memory device, among other causes) are reduced.

Advantageously, the automated blood sampling and infusion system as disclosed herein facilitates reduction of such risks by providing an automated and accurate time management for both dose administration (for example, with a smart device controlling the infusion pump) and blood sampling (as an illustrative example, as few as 3 samples or as many as 12 samples, or any other sample number therebetween, obtained within 6 hours after infusion at precisely-measured time increments), as well as an automated and accurate dosage management based on sampling a small volume of blood and measuring the drug concentration therein. The small volume sample (for example, between 0.1 mL and 0.5 mL, between 0.5 mL and 1.5 mL, between 1.5 mL and 2.5 mL, or any other suitable value or range therebetween) facilitates more frequent drug concentration measurement to take place, as well as reducing the burden on the patient of drawing a large quantity of blood for sampling. As previously mentioned, the smart device may implement processes (which may be performed by algorithms such as computer program codes stored in the memory unit and run by the processor, for example) to adjust the dosage parameters and "learn" from real-world experience beyond the clinical trials. Improved accuracy and precision can lead to benefits not just for doctors (who can now have better data points to make more informed decisions on diagnosis or prognosis, as well as being able to make faster decisions to avoid complications due to a delayed response) and the patients (who can avoid acute and chronic unintended consequences of human errors), but also hospital administration (who can improve safety and empty beds faster with a better patient discharge rate), medical insurance providers (who can reduce risks and improve efficacy as well as avoiding adverse drug reactions by patients), nurses (who can reduce the number of blood redraws and resampling with simplified and higher-quality time-tracked sampling), clinical laboratories (who can reduce the turnaround time with drug and biomarker concentrations), clinical pharmacologists (who can use results with better accuracy for their clinical researches), pharmaceutical companies (who can extend clinical trial principles to actual care in order to potentially reduce the impact of highly variable pharmacokinetics or pharmacodynamics on drug success), contract research organizations (who can perform better modeling with richer pharmacokinetics and pharmacodynamics data being provided early on, as well as reducing labor and improving sample data quality), and the FDA (who can make better decisions with richer data from preclinical and clinical studies with regards to drug approval, as well as avoiding the impact of chronic organ toxicity over a prolonged period of exposure if the dose can be adjusted appropriately).

In some examples, the sensors are capable of sensing or measuring at least one of: blood temperature, blood pressure, pulse (heart rate), or breathing rate (respiratory rate). In some examples, the sensors can measure the amount or level of gases in the patient's blood. These measurements can be accurately plotted along a time axis. Other measurements include flow rate of the fluid (for sampling and/or infusion), volume of the fluid being transported via the conduits (for sampling and/or infusion), concentration of the fluid (mainly for infusion), etc.

There are numerous advantages provided by precise time recording of each sample collection event and infusion event. In some examples, keeping an accurate, real-time record of the events reduces the likelihood of human error caused by carelessness, negligence, or malpractice. Accurate records also allow doctors and nurses to determine a possible cause of a problem when the patient experiences a change in health condition, for example due to overdosing, underdosing, or side effects of the drug infusion. For example, when there are multiple drugs that are being infused at different times, the specific time of infusion for each of the drugs is a key to identify which of the drugs may have triggered such change in the health condition of the patient. Furthermore, integrating the infusion with the blood sampling may improve safety by confirming the amount and type of the fluid.

Allowing the system to automatically perform blood sampling and infusion can also avoid wasting blood that is collected (for example, when blood collected at a wrong time interval would be less useful and the sample may need to be redrawn or collected again), avoid anemia caused by oversampling blood by hand, avoid hemolysis caused by a too aggressive manual pull on a catheter, avoid dilution of the blood sample by inadequate clearing of lock solution from the catheter before sample collection, and/or reduce the risk of infection (e.g., as caused by blood-borne pathogens) resulting from conventional blood sampling procedures performed by hand. In some examples, the catheter patency may be automated such that any obstruction in the catheter is avoided or detected by the sensors. Automating these processes which would otherwise have to be performed by doctors and nurses reduces the workload on these doctors and nurses, especially when there are multiple patients to look after and each one of the patients has unique needs that may be difficult for the doctors and nurses to track and remember, or when the hospital or clinic is understaffed and the doctors and nurses may be distracted when performing sample collection and infusion, leading to increased risk of errors.

## Claims

1. An automated blood sampling and infusion system (100) comprising:
a sampling pump (300) in communication with a blood sample collection apparatus (606);
an infusion pump (300) configured to perform infusion; and
a controller (106) configured to:
control the infusion pump (300) to perform the infusion on a patient during an infusion event; control the sampling pump (300) to draw a plurality of unique blood samples from the patient into a corresponding plurality of sample containers (608) of the blood sample collection apparatus (606) during a plurality of sample collection events following the infusion event; and automatically store in a memory unit (202) a timestamped data of the infusion event, the timestamped data including times each of the plurality of sample collection events occurred.

2. The system of claim 1, wherein the timestamped data includes (a) a start time of each of the plurality of sample collection events or the infusion event and (b) an end time of each of the plurality of sample collection events or the infusion event.

3. The system of claim 1 or 2, further comprising a user interface (204, 308) to input information regarding the plurality of sample collection events and the infusion event.

4. The system of claim 1, wherein an amount of blood drawn for the sample ranges from 0.01 cm³ (cc) to 10 cm³ (cc).

5. The system of claim 4, further comprising a user interface (204, 308) to facilitate inputting the amount of blood to be drawn for the sample.

6. The system of any one of claims 1-5, further comprising at least one additional electronic device (206) operatively coupled with the controller (106) via a wired or wireless network connection (108), the at least one additional electronic device (206) configured to provide information regarding the plurality of sample collection events and the infusion event and to receive the timestamped data of each of the plurality of sample collection events and infusion event facilitated by the controller (106).

7. The system of any one of claims 1-6, further comprising a multi-lumen catheter (702) comprising at least a first lumen and a second lumen, wherein the first lumen is in fluid communication with an infusion solution storage (306) and the second lumen is in fluid communication with a sample collection storage (304).

8. The system of any one of claims 1-7, wherein the memory unit (202) stores therein a drug library comprising instructions on the plurality of sample collection events and the infusion event associated with each of a plurality of drugs that may be administered during the infusion event.

9. The system of any one of claims 1-8, wherein the controller (106) is configured to control the sampling pump (300) to perform the plurality of sample collection events associated with the timestamped data, and
wherein the blood sample collection apparatus (606) includes a cassette removably installed therein and containing the plurality of sample containers (608), and each of the plurality of sample containers (608) contains one sample of the blood drawn from the patient during one of the plurality of sample collection events.

10. The system of claim 9, wherein the blood sample collection apparatus (606) is operable to refrigerate the plurality of sample containers (608) during the plurality of sample collection events.

11. The system of claim 9 or 10, wherein an amount of blood drawn for the sample ranges from 0.01 cm³ (cc) to 10 cm³ (cc).

12. The system of any one of claims 9-11, further comprising a user interface (204, 308) to facilitate inputting an amount of blood to be drawn for the sample and a time interval between the plurality of sample collection events.

13. An automated blood sampling and infusion system (100) comprising:
a sampling pump (300) in communication with a blood sample collection apparatus (606) containing a plurality of sample containers (608), each of the sample containers (608) configured to contain one sample of blood drawn from a patient during one of a plurality of sample collection events;
an infusion pump (300) configured to perform infusion during a plurality of infusion events;
a user interface (204, 308) configured to facilitate inputting an amount of blood to be drawn for each of the samples and a time interval between the sample collection events;
a controller (106) configured to:
control the infusion pump (300) to perform the infusion on the patient during one of the infusion events;
control, based on the amount of blood to be drawn and the time interval that are inputted, the sampling pump (300) to draw the blood from the patient into the blood sample collection apparatus (606) during one of the sample collection events following the infusion event; and
automatically store in a memory unit (202) a timestamped data of the infusion event, the timestamped data including times each of the plurality of sample collection events occurred.

14. The system of claim 13, wherein the amount of blood drawn for the sample ranges from 0.01 cm³ (cc) to 10 cm³ (cc).

## Patentansprüche

1. Automatisiertes Blutentnahme- und Infusionssystem (100), das Folgendes umfasst:
eine Entnahmepumpe (300) in Kommunikation mit einer Blutprobensammeleinrichtung (606);
eine Infusionspumpe (300), die dazu ausgelegt ist, eine Infusion durchzuführen; und
eine Steuerung (106), die zu Folgendem ausgelegt ist:
Steuern der Infusionspumpe (300) zum Durchführen der Infusion an einem Patienten während eines Infusionsereignisses;
Steuern der Entnahmepumpe (300) zum Absaugen einer Vielzahl von eindeutigen Blutproben vom Patienten in eine entsprechende Vielzahl von Probenbehältern (608) der Blutprobensammeleinrichtung (606) während einer Vielzahl von Probensammelereignissen nach dem Infusionsereignis; und
automatisches Speichern von Zeitstempeldaten des Infusionsereignisses in einer Speichereinheit (202), wobei die Zeitstempeldaten Zeiten beinhalten, zu denen die Vielzahl von Probensammelereignissen erfolgte.

2. System nach Anspruch 1, wobei die Zeitstempeldaten (a) eine Startzeit von jedem der Vielzahl von Probensammelereignissen oder des Infusionsereignisses und (b) eine Endzeit von jedem der Vielzahl von Probensammelereignissen oder des Infusionsereignisses beinhalten.

3. System nach Anspruch 1 oder 2, das ferner eine Benutzerschnittstelle (204, 308) zum Eingeben von Informationen, die sich auf die Vielzahl von Probensammelereignissen und das Infusionsereignis beziehen, umfasst.

4. System nach Anspruch 1, wobei eine Menge von für die Probe abgesaugtes Blut von 0,01 cm³ (cc) bis 10 cm³ (cc) liegt.

5. System nach Anspruch 4, das ferner eine Benutzerschnittstelle (204, 308) zum Ermöglichen des Eingebens der Menge des für die Probe abzusaugendes Blutes umfasst.

6. System nach einem der Ansprüche 1 bis 5, das mindestens eine zusätzliche elektronische Vorrichtung (206) umfasst, die via eine drahtgebundene oder drahtlose Netzwerkverbindung (108) an die Steuerung (106) wirkgekoppelt ist, wobei die mindestens eine zusätzliche elektronische Vorrichtung (206) dazu ausgelegt ist, Informationen bereitzustellen, die sich auf die Vielzahl von Probensammelereignissen und das Infusionsereignis beziehen, und die Zeitstempeldaten von jedem der Vielzahl von Probensammelereignissen und dem Infusionsereignis zu empfangen, was von der Steuerung (106) ermöglicht wird.

7. System nach einem der Ansprüche 1 bis 6, das ferner einen Multilumenkatheter (702) umfasst, der mindestens ein erstes Lumen und ein zweites Lumen umfasst, wobei das erste Lumen mit einem Infusionslösungsspeicher (306) in Fluidkommunikation steht und das zweite Lumen mit einem Probensammelspeicher (304) in Fluidkommunikation steht.

8. System nach einem der Ansprüche 1 bis 7, wobei in der Speichereinheit (202) eine Medikamentenbibliothek gespeichert ist, die Anweisungen über die Vielzahl von Probensammelereignissen und das Infusionsereignis umfasst, die mit jedem einer Vielzahl von Medikamenten verknüpft sind, die während des Infusionsereignisses verabreicht werden können.

9. System nach einem der Ansprüche 1 bis 8, wobei die Steuerung (106) dazu ausgelegt ist, die Entnahmepumpe (300) zum Durchführen der Vielzahl von Probensammelereignissen durchzuführen, die mit den Zeitstempeldaten verknüpft sind, und
wobei die Blutprobensammeleinrichtung (606) eine Kassette beinhaltet, die entfernbar darin installiert ist und die Vielzahl von Probenbehältern (608) enthält, und jeder der Vielzahl von Probenbehältern (608) eine Probe des Blutes enthält, das vom Patienten während eines der Vielzahl von Probensammelereignissen abgesaugt wird.

10. System nach Anspruch 9, wobei die Blutprobensammeleinrichtung (606) zum Kühlen der Vielzahl von Probenbehältern (608) während der Vielzahl von Probensammelereignissen betreibbar ist.

11. System nach Anspruch 9 oder 10, wobei eine Menge von für die Probe abgesaugtes Blut von 0,01 cm³ (cc) bis 10 cm³ (cc) liegt.

12. System nach einem der Ansprüche 9 bis 11, das ferner eine Benutzerschnittstelle (204, 308) zum Ermöglichen des Eingebens einer Menge von für die Probe abzusaugendes Blut und eines Zeitintervalls zwischen der Vielzahl von Probensammelereignissen umfasst.

13. Automatisiertes Blutentnahme- und Infusionssystem (100), das Folgendes umfasst:
eine Entnahmepumpe (300), die mit einer Blutprobensammeleinrichtung (606) in Kommunikation steht, die eine Vielzahl von Probenbehältern (608) enthält, wobei jeder der Probenbehälter (608) dazu ausgelegt ist, eine Probe von von einem Patienten während einer Vielzahl von Probensammelereignissen abzusaugenden Blut zu enthalten;
eine Infusionspumpe (300), die dazu ausgelegt ist, während einer Vielzahl von Infusionsereignissen eine Infusion durchzuführen;
eine Benutzerschnittstelle (204, 308), die zum Ermöglichen des Eingebens einer Menge von für jede der Proben abzusaugendem Blut und des Zeitintervalls zwischen den Probensammelereignissen ausgelegt ist;
eine Steuerung (106), die zu Folgendem ausgelegt ist:
Steuern der Infusionspumpe (300) zum Durchführen der Infusion am Patienten während eines der Infusionsereignisse;
Steuern der Entnahmepumpe (300) zum Absaugen des Blutes vom Patienten in die Blutprobensammeleinrichtung (606) während eines der Probensammelereignisse nach dem Infusionsereignis auf Basis der Menge von abzusaugendem Blut und des Zeitintervalls, die eingegeben werden; und
automatisches Speichern von Zeitstempeldaten des Infusionsereignisses in einer Speichereinheit (202), wobei die Zeitstempeldaten Zeiten beinhalten, zu denen die Vielzahl von Probensammelereignissen erfolgte.

14. System nach Anspruch 13, wobei die Menge des für die Probe abzusaugenden Blutes von 0,01 cm³ (cc) bis 10 cm³ (cc) liegt.

## Revendications

1. Système automatisé de prélèvement sanguin et de perfusion (100) comprenant :
une pompe de prélèvement (300) en communication avec un appareil de collecte d'échantillon sanguin (606) ;
une pompe de perfusion (300) configurée pour effectuer une perfusion ; et
un contrôleur (106) configuré pour :
commander la pompe de perfusion (300) pour effectuer la perfusion sur un patient au cours d'un événement de perfusion ; commander la pompe de prélèvement (300) pour prendre une pluralité d'échantillons de sang uniques du patient dans une pluralité de conteneurs d'échantillons (608) correspondants de l'appareil de collecte d'échantillon sanguin (606) pendant une pluralité d'événements de collecte d'échantillons suivant l'événement de perfusion ; et stocker automatiquement dans une unité de mémoire (202) des données horodatées de l'événement de perfusion, les données horodatées comportant les heures auxquelles la pluralité d'événements de collecte d'échantillons se sont produits.

2. Système selon la revendication 1, dans lequel les données horodatées comportent (a) une heure de début de chacun de la pluralité d'événements de collecte d'échantillons ou de l'événement de perfusion et (b) une heure de fin de chacun de la pluralité d'événements de collecte d'échantillons ou de l'événement de perfusion.

3. Système selon la revendication 1 ou 2, comprenant en outre une interface utilisateur (204, 308) pour entrer des informations concernant la pluralité d'événements de collecte d'échantillons et l'événement de perfusion.

4. Système selon la revendication 1, dans lequel une quantité de la prise de sang pour l'échantillon va de 0,01 cm³(cc) à 10 cm³(cc).

5. Système selon la revendication 4, comprenant en outre une interface utilisateur (204, 308) pour faciliter l'entrée de la quantité de la prise de sang à faire pour l'échantillon.

6. Système selon l'une des revendications 1 à 5, comprenant en outre au moins un dispositif électronique (206) supplémentaire couplé de manière opérationnelle au contrôleur (106) via une connexion réseau filaire ou sans fil (108), l'au moins un dispositif électronique (206) supplémentaire étant configuré pour fournir des informations concernant la pluralité d'événements de collecte d'échantillons et l'événement de perfusion, et pour recevoir les données horodatées de chacun de la pluralité d'événements de collecte d'échantillons et de l'événement de perfusion facilités par le contrôleur (106).

7. Système selon l'une des revendications 1 à 6, comprenant en outre un cathéter multi-lumières (702) comprenant au moins une première lumière et une deuxième lumière, dans lequel la première lumière est en communication fluidique avec un stockage de solution de perfusion (306) et la deuxième lumière est en communication fluidique avec un stockage de collecte d'échantillons (304).

8. Système selon l'une des revendications 1 à 7, dans lequel l'unité de mémoire (202) stocke une pharmacothèque comprenant des instructions sur la pluralité d'événements de collecte d'échantillons et l'événement de perfusion associés à chacun d'une pluralité de médicaments pouvant être administrés pendant l'événement de perfusion.

9. Système selon l'une des revendications 1 à 8, dans lequel le contrôleur (106) est configuré pour commander la pompe de prélèvement (300) pour effectuer la pluralité d'événements de collecte d'échantillons associés aux données horodatées, et
dans lequel l'appareil de collecte d'échantillon sanguin (606) comporte une cassette installée de manière amovible à l'intérieur et contenant la pluralité de conteneurs d'échantillons (608), et chacun de la pluralité de conteneurs d'échantillons (608) contient un échantillon de la prise de sang du patient pendant l'un de la pluralité d'événements de collecte d'échantillons.

10. Système selon la revendication 9, dans lequel l'appareil de collecte d'échantillon sanguin (606) permet de réfrigérer la pluralité de conteneurs d'échantillons (608) pendant la pluralité d'événements de collecte d'échantillons.

11. Système selon la revendication 9 ou 10, dans lequel une quantité de la prise de sang pour l'échantillon va de 0,01 cm³(cc) à 10 cm³(cc).

12. Système selon l'une des revendications 9 à 11, comprenant en outre une interface utilisateur (204, 308) pour faciliter l'entrée d'une quantité de la prise de sang à faire pour l'échantillon et un intervalle de temps entre la pluralité d'événements de collecte d'échantillons.

13. Système automatisé de prélèvement sanguin et de perfusion (100) comprenant :
une pompe de prélèvement (300) en communication avec un appareil de collecte d'échantillon sanguin (606) contenant une pluralité de conteneurs d'échantillons (608), chacun des conteneurs d'échantillons (608) étant configuré pour contenir un échantillon de la prise de sang d'un patient pendant l'un d'une pluralité d'événements de collecte d'échantillons ;
une pompe de perfusion (300) configurée pour effectuer une perfusion pendant une pluralité d'événements de perfusion ;
une interface utilisateur (204, 308) configurée pour faciliter l'entrée d'une quantité de la prise de sang à faire pour chacun des échantillons et un intervalle de temps entre les événements de collecte d'échantillons ;
un contrôleur (106) configuré pour :
commander la pompe de perfusion (300) pour effectuer la perfusion sur le patient pendant l'un des événements de perfusion ;
sur la base de la quantité de la prise de sang à faire et de l'intervalle de temps qui sont entrés, commander la pompe de prélèvement (300) pour prendre le sang du patient dans l'appareil de collecte d'échantillon sanguin (606) pendant l'un des événements de collecte d'échantillons suivant l'événement de perfusion ; et
stocker automatiquement dans une unité de mémoire (202) des données horodatées de l'événement de perfusion, les données horodatées comportant les heures auxquelles chacun de la pluralité d'événements de collecte d'échantillons s'est produit.

14. Système selon la revendication 13, dans lequel la quantité de la prise de sang pour l'échantillon va de 0,01 cm³(cc) à 10 cm³(cc).
